(19) Europäisches Patentamt
European Patent Office
Office européen des brevets

(11) Veröffentlichungsnummer: **0 108 236**
**A2**

(12) **EUROPÄISCHE PATENTANMELDUNG**

(21) Anmeldenummer: **83109664.9**

(22) Anmeldetag: **28.09.83**

(51) Int. Cl.³: **C 07 D 249/08**
C 07 D 233/58, A 01 N 43/64
A 01 N 43/50

(30) Priorität: **09.10.82 DE 3237476**

(43) Veröffentlichungstag der Anmeldung:
**16.05.84 Patentblatt 84/20**

(84) Benannte Vertragsstaaten:
**BE CH DE FR GB LI**

(71) Anmelder: **BAYER AG**
**Konzernverwaltung RP Patentabteilung**
**D-5090 Leverkusen 1 Bayerwerk(DE)**

(72) Erfinder: **Draber, Wilfried, Dr.**
**In den Birken 81**
**D-5600 Wuppertal 1(DE)**

(72) Erfinder: **Büchel, Karl Heinz, Prof. Dr.**
**Dabringhausenerstrasse 42**
**D-5093 Burscheid(DE)**

(72) Erfinder: **Reinecke, Paul, Dr.**
**Lessingstrasse 11**
**D-5090 Leverkusen 3(DE)**

(54) **1-Aryl-3-azolyl-4,4-dimethyl-1-pentene.**

(57) Die Erfindung betrifft neue 1-Aryl-3-azolyl-4,4-dimethyl-1-pentene der allgemeinen Formel (I)

$$Ar-CH=CH-\underset{\displaystyle AZ}{\underset{|}{CH}}-C(CH_3)_3 \qquad (I)$$

in welcher
Ar für gegebenenfalls substituiertes Aryl steht und
Az für 1,2,4-Triazol-1-yl oder Imidazol-1-yl steht.

Sie werden erhalten, wenn man 1-Aryl-3-halogen-4,4-dimethyl-1-pentene der allgemeinen Formel (II)

$$Ar-CH=CH-\underset{\displaystyle Hal}{\underset{|}{CH}}-C(CH_3)_3 \qquad (II)$$

in welcher
Ar die obige Bedeutung hat und Hal für Halogen steht, mit 1,2,4-Triazol oder Imidazol gegebenenfalls in Gegenwart eines Verdünnungsmittels und gegebenenfalls in Gegenwart eines Säurebindemittels umsetzt.

Die neuen 1-Aryl-3-azolyl-4,4-dimethyl-1-pentene der allgemeinen Formel (I) sind als Pflanzenschutzmittel geeignet, insbesondere als Fungizide.

EP 0 108 236 A2

BAYER AKTIENGESELLSCHAFT 5090 Leverkusen, Bayerwerk

Zentralbereich
Patente, Marken und Lizenzen Slr/Bas/ABc
Ia

## 1-Aryl-3-azolyl-4,4-dimethyl-1-pentene

Die Erfindung betrifft neue 1-Aryl-3-azolyl-4,4-dimethyl-1-pentene, ein Verfahren zu ihrer Herstellung sowie ihre Verwendung als Fungizide.

Es ist bereits bekannt, daß Azolyl-substituierte Alkenderivate, wie z.B. 4-(1,2,4-Triazol-1-yl)-2,6,6-trimethyl-2-hepten-5-on fungizide Eigenschaften besitzen (vgl. DE-OS 29 05 981). Die Wirkung dieser Verbindungen ist jedoch insbesondere bei niedrigen Aufwandmengen und -konzentrationen nicht immer voll befriedigend.

Es wurden neue 1-Aryl-3-azolyl-4,4-dimethyl-1-pentene der allgemeinen Formel (I)

$$Ar-CH=CH-\underset{\displaystyle Az}{\underset{|}{CH}}-C(CH_3)_3 \qquad (I)$$

in welcher

Ar für gegebenenfalls substituiertes Aryl steht und

Az für 1,2,4-Triazol-1-yl oder Imidazol-1-yl steht,

sowie deren pflanzenverträgliche Säureadditionssalze und Metallsalz-Komplexe gefunden.

Die Verbindungen der Formel (I) können in verschiedenen geometrischen Isomerenformen (cis-trans-Isomerie) vorliegen. Die Formel (I) umfaßt sowohl die Isomeren als auch deren Gemische.

Weiterhin wurde gefunden, daß man 1-Aryl-3-azolyl-4,4-dimethyl-1-pentene der allgemeinen Formel (I)

$$\underset{\displaystyle\quad\quad\quad\quad\quad\quad\quad\quad\quad Az}{Ar\text{-}CH{=}CH\text{-}CH\text{-}C(CH_3)_3} \qquad (I)$$

in welcher
Ar für gegebenenfalls substituiertes Aryl steht und

Az für 1,2,4-Triazol-1-yl oder Imidazol-1-yl steht,

erhält, wenn man 1-Aryl-3-halogen-4,4-dimethyl-1-pentene der allgemeinen Formel (II),

$$\underset{\displaystyle\quad\quad\quad\quad\quad\quad\quad\quad\quad Hal}{Ar\text{-}CH{=}CH\text{-}CH\text{-}C(CH_3)_3} \qquad (II)$$

in welcher

Ar die oben angegebene Bedeutung hat und

Hal für Halogen steht,

mit 1,2,4-Triazol oder Imidazol gegebenenfalls in Gegenwart eines Verdünnungsmittels und gegebenenfalls in Gegegenwart eines Säurebindemittels umsetzt.

An die so erhaltenen Verbindungen der allgemeinen Formel (I) kann gegebenenfalls eine Säure oder ein Metallsalz addiert werden.

Schließlich wurde gefunden, daß die neuen 1-Aryl-3-azolyl-4,4-dimethyl-1-pentene der allgemeinen Formel (I) sowie deren Säureadditionssalze und Metallsalz-Komplexe fungizide Eigenschaften aufweisen.

Überraschenderweise zeigen die erfindungsgemäßen Verbindungen der allgemeinen Formel (I) eine bessere fungizide Wirksamkeit als die aus dem Stand der Technik bekannten Azolyl-substituierten Alkenderivate, wie z.B. das 4-(1,2,4-Triazol-1-yl)-2,6,6-trimethyl-2-hepten-5-on, welche chemisch und wirkungsmäßig naheliegende Verbindungen sind. Die erfindungsgemäßen Substanzen stellen somit eine wertvolle Bereicherung der Technik dar.

Die erfindungsgemäßen 1-Aryl-3-azolyl-4,4-dimethyl-1-pentene sind durch die Formel (I) allgemein definiert.

Bevorzugt sind diejenigen Verbindungen der allgemeinen Formel (I), bei denen

Az für 1,2,4-Triazol-1-yl oder Imidazol-1-yl steht und

Ar für gegebenenfalls einfach oder mehrfach, gleich oder verschieden substituiertes Aryl mit 6 bis 10 Kohlenstoffatomen steht, wobei als Substituenten in Frage kommen: Halogen, Cyano, Alkyl, Alkoxy, Alkylthio, Mono- und Dialkylamino mit jeweils 1 bis 4 Kohlenstoffatomen in den jeweiligen Alkylteilen, Dioxyalkylen mit 1 bis 2 Kohlenstoffatomen, sowie gegebenenfalls durch Alkyl mit 1 bis 4 Kohlenstoffatomen substituiertes 5- oder 6-gliedriges Heteroaryl mit 1 bis 3 Heteroatomen, insbesondere Stickstoff, Sauerstoff und/oder Schwefel.

Besonders bevorzugt sind Verbindungen der Formel (I), bei denen

Az für 1,2,4-Triazol-1-yl oder Imidazol-1-yl steht und

Ar für gegegebenenfalls ein- bis dreifach, gleich oder verschieden substituiertes Phenyl oder Naphthyl steht, wobei als Substituenten insbesondere in Frage kommen: Fluor, Chlor, Brom, Methyl, Ethyl, Methoxy, Methylthio, Methylamino und Dimethylamino, Dioxymethylen sowie durch Methyl und/oder Ethyl substituiertes 1,2,3-Triazol-2-yl.

Im einzelnen seien außer den bei den Herstellungsbeispielen genannten Verbindungen die folgenden Verbindungen der allgemeinen Formel (I) aufgeführt:

$$Ar - CH=CH - \underset{\underset{Az}{|}}{CH} - C(CH_3)_3 \qquad (I)$$

| Ar | Az |
|---|---|
| 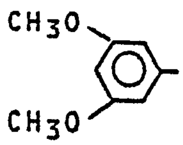 | 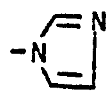 |
| 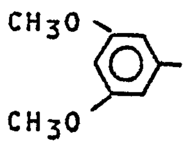 | 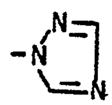 |
| 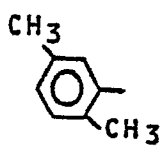 | 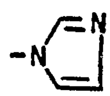 |
| 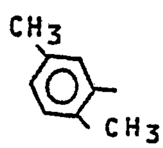 | 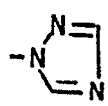 |
| 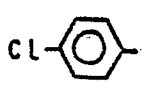 | 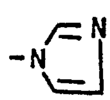 |

| Ar | Az |
|---|---|
| 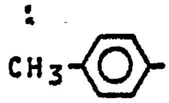 | 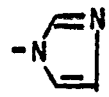 |
| 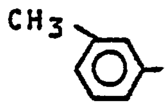 | 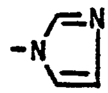 |
| 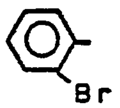 | 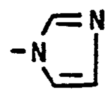 |
| 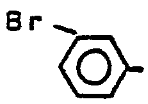 | 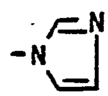 |
| 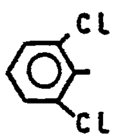 | 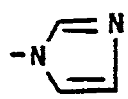 |
| 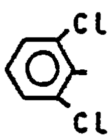 | 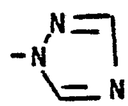 |
| 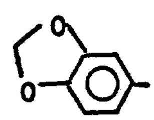 | 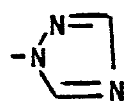 |
| 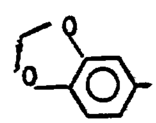 | 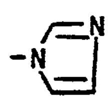 |
| 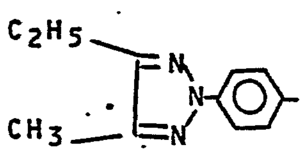 | 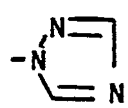 |
| 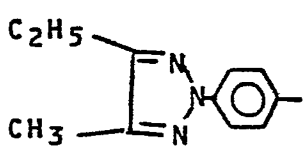 | 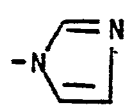 |

Verwendet man beispielsweise 1-(2,6-Dichlorphenyl)-3-chlor-4,4-dimethyl-1-penten und 1,2,4-Triazol als Ausgangsstoffe, so kann der Reaktionsablauf des erfindungsgemäßen Verfahrens durch das folgende Formelschema wiedergegeben werden:

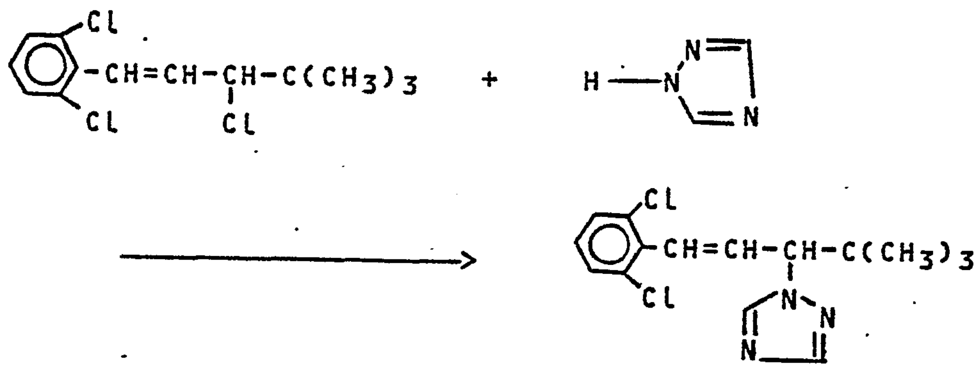

Die zur Durchführung des erfindungsgemäßen Verfahrens als Ausgangsstoffe benötigten 1-Aryl-3-halogen-4,4-dimethyl-1-pentene sind durch die Formel (II) allgemein definiert.

Die 1-Aryl-3-halogen-4,4-dimethyl-1-pentene der allgemeinen Formel (II) sind noch nicht bekannt. Sie können nach bekannten Verfahren erhalten werden, indem man zunächst in prinzipiell bekannter Weise aromatische Aldeyhde der Formel (III)

$$Ar - \underset{\overset{\|}{O}}{C} - H \qquad (III)$$

in welcher

Ar die oben angegebene Bedeutung hat,

mit Pinakolon in Gegenwart eines Verdünnungsmittels, wie z.B. Ethanol und in Gegenwart einer Base, wie z.B. Natriumhydroxid bei 20°C bis 30°C zu ungesättigten Ketonen der allgemeinen Formel (IV) kondensiert,

$$Ar-CH=CH-\underset{\underset{O}{\|}}{C}-C(CH_3)_3 \qquad (IV)$$

in welcher

Ar die oben angegebene Bedeutung hat,

und diese in einer 2. Stufe in ebenfalls prinzipiell bekannter Weise mit einem komplexen Hydrid, wie z.B. Natrium- oder Calciumborhydrid in Gegenwart eines Verdünnungsmittels, wie z.B. Methanol, bei Temperaturen zwischen 0°C und 10°C zu den ungesättigten Alkoholen der allgemeinen Formel (V) reduziert

$$Ar-CH=CH-\underset{\underset{OH}{|}}{CH}-C(CH_3)_3 \qquad (V)$$

in welcher

Ar die oben angegebene Bedeutung hat,

und diese in einer 3. Stufe in ebenfalls prinzipiell bekannter Weise mit einem Halogenierungsmittel, wie z.B. Thionylchlorid, gegebenenfalls in Gegenwart eines Verdünnungsmittels, wie z.B. Dichlormethan, bei Temperaturen zwischen 35°C und 80°C substituiert.

Die aromatischen Aldehyde der Formel (III) sind größtenteils allgemein bekannte Verbindungen der organischen Chemie.

Der Aldehyd der Formel (IIIa)

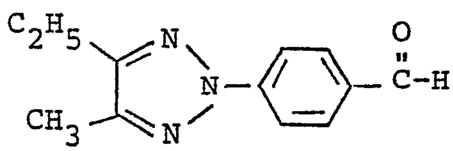

(IIIa)

ist bekannt aus der DE-OS 1 962 353.

Als Verdünnungsmittel kommen für das erfindungsgemäße Verfahren inerte organische Lösungsmittel in Frage. Hierzu gehören vorzugsweise chlorierte Kohlenwasserstoffe, wie z.B. Dichlormethan, Chloroform, Tetrachlorkohlenstoff oder Chlorbenzol, Amide, wie Dimethylformamid, Dimethylacetamid oder N-Methylformanilid, Nitrile, wie Acetonitril oder Propionitril, sowie die hochpolaren Lösungsmittel Dimethylsulfoxid, Sulfolan oder Hexamethylphosphorsäuretriamid.

Das erfindungsgemäße Verfahren kann in Gegenwart eines Säurebindemittels vorgenommen werden. Man kann alle

üblicherweise verwendbaren anorganischen oder organischen Säurebinder zugeben, wie Alkalicarbonate, z.B. Natriumcarbonat und Kaliumcarbonat, wie niedere tertiäre Alkylamine, Cycloalkylamine oder Aralkylamine, z.B. Triethylamin, N,N-Dimethylcyclohexylamin und N,N-Dimethylbenzylamin. Ebenfalls möglich ist ein entsprechender Überschuß an der Triazol- bzw. Imidazol-Komponente.

Die Reaktionstemperaturen können bei dem erfindungsgemäßen Verfahren in einem größeren Bereich variiert werden. Im allgemeinen arbeitet man zwischen 0°C und 150°C, vorzugsweise zwischen 35 und 90°C.

Zur Durchführung des erfindungsgemäßen Verfahrens setzt man auf 1 Mol der Verbindungen der allgemeinen Formel (II) vorzugsweise 1 bis 3 Mol 1,2,4-Triazol bzw. Imidazol ein. Die Isolierung der Verbindungen der allgemeinen Formel (I) erfolgt nach üblichen Methoden.

Gemäß einer besonderen Ausführungsform des erfindungsgemäßen Verfahrens kann auch so vorgegangen werden, daß man zunächst die Zwischenprodukte der allgemeinen Formel (II) herstellt und ohne deren Isolierung die weitere Umsetzung durchführt und so die Endprodukte der allgemeinen Formel (I) im Rahmen eines 'Eintopfverfahrens' in einem Arbeitsgang erhält.

Zur Herstellung von physiologisch verträglichen Säureadditionssalzen der Verbindungen der allgemeinen Formel (I) kommen vorzugsweise folgende Säuren in Frage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, sowie Sulfonsäuren, wie z.B. p-Toluolsulfonsäure und 1,5-Naphthalin-disulfonsäure.

Die Säureadditionssalze der Verbindungen der allgemeinen Formel (I) können in einfacher Weise nach üblichen Salzbildungsmethoden, z.B. durch Lösen einer Verbindung der allgemeinen Formel (I) in einem geeigneten organischen Lösungsmittel und Hinzufügen der Säure, z.B. Chlorwasserstoffsäure, erhalten werden und in bekannter Weise, durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. Chlorwasserstoffsäure und Bromwasserstoffsäure, ferner Phosphorsäure, Salpetersäure und Schwefelsäure.

Die Metallsalz-Komplexe von Verbindungen der allgemeinen Formel (I) können in einfacher Weise nach üblichen Verfahren erhalten werden, so z.B. durch Lösen des Metallsalzes in Alkohol, wie z.B. Ethanol und Hinzufügen zur Verbindung der allgemeinen Formel (I). Man kann die Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie z.B. gegen Pyrenophora teres,

sowie gegen den Erreger des Getreidemehltaus (Erysiphe graminis) gegen Septoria nodorum und gegen Cochliobolus sativus eingesetzt werden. Daneben besitzen die erfindungsgemäßen Wirkstoffe auch eine gute Wirkung gegen den Erreger des Apfelschorfs (Venturia inaequalis) und gegen Erreger von Reiskrankheiten, z.B. Pyricularia oryzae und Pellicularia sasakii.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen,

Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

## Herstellungsbeispiele

### Beispiel 1

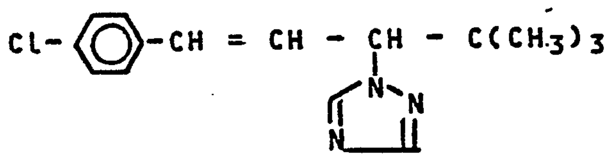

12 g (0,053 Mol) 1-(4-Chlorphenyl)-4,4-dimethyl-1-penten-3-ol werden in 100 ml Dichlormethan gelöst und tropfenweise mit 8 g (0,067 Mol) Thionylchlorid versetzt. Nach beendeter Zugabe kocht man zwei Stunden am Rückfluß, engt die Reaktionslösung im Vakuum ein und nimmt den öligen Rückstand in 50 ml Acetonitril auf. Nach Zugabe von 11 g (0,15 Mol) 1,2,4-Triazol kocht man weitere 12 Stunden am Rückfluß, entfernt das Lösungsmittel im Vakuum, nimmt den Rückstand in 100 ml Dichlormethan auf, wäscht mehrfach mit Wasser, trocknet über Natriumsulfat und entfernt das Lösungsmittel im Vakuum. Man erhält 8,5 g (71 % der Theorie) an 1-(4-Chlorphenyl)-4,4-dimethyl-3-(1,2,4-triazol-1-yl)-1-penten vom Schmelzpunkt 115°C-117°C.

### Herstellung des Ausgangsproduktes

a)

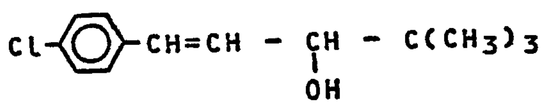

22,2 g (0,1 Mol) 1-(4-Chlorphenyl)-4,4-dimethyl-1-penten-3-on in 50 ml Methanol werden bei 0°C bis 20°C mit 4 g (0,1 Mol) Natriumborhydrid versetzt. Nach beendeter Zugabe rührt man 3 Stunden bei 20°C bis 25°C. Zur Aufarbeitung wird das Lösungsmittel im Vakuum entfernt, der Rückstand in Dichlormethan und Wasser aufgenommen und mit Essigsäure neutralisiert. Man trennt die organische Phase ab, wäscht sie mehrmals mit Wasser, trocknet sie über Natriumsulfat und entfernt das Lösungsmittel im Vakuum. Man erhält 20,3 g (90 % der Theorie) an 1-(4-Chlorphenyl)-4,4-dimethyl-1-penten-3-ol vom Schmelzpunkt 40-43°C.

b) 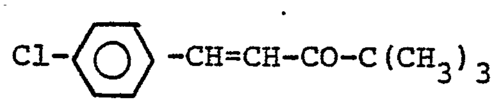

140,5 g (1 Mol) p-Chlorbenzaldehyd, 100 g (1 Mol) Pinakolon und 4 g (0,1 Mol) Natriumhydroxid werden in 300 ml Ethanol 12 Stunden am Rückfluß gekocht. Nach dem Abkühlen wird filtriert, das Filtrat im Vakuum eingeengt, der ölige Rückstand in Dichlormethan/Wasser aufgenommen und mit Essigsäure neutralisiert. Die organische Phase wird mehrfach mit Wasser gewaschen, über Natriumsulfat getrocknet und im Vakuum eingedampft. Man erhält 131,7 g (59 % der Theorie) an 1-(4-Chlorphenyl)-4,4-dimethyl-1-penten-3-on vom Schmelzpunkt 84-87°C

In entsprechender Weise erhält man die folgenden Verbindungen der allgemeinen Formel (I)

$$\underset{\substack{| \\ Az}}{Ar-CH=CH-CH-C(CH_3)_3} \qquad (I)$$

| Bsp. Nr. | Ar | Az | Schmelzpunkt [°C], Brechungsindex [$n_D^{20}$], Siedepkt. [°C/mm Hg] |
|---|---|---|---|
| 2 | 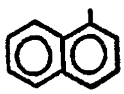 | 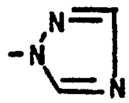 | 114-116 |
| 3 | 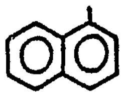 | 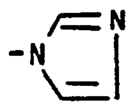 | 151-154 |
| 4 | 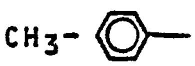 | 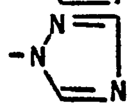 | 1,5414 |
| 5 | 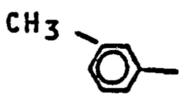 | 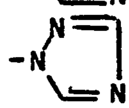 | 150/0,1 |
| 6 | 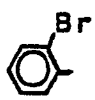 | 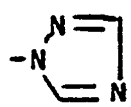 | 173/0,1 |
| 7 | 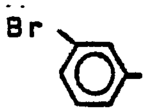 | 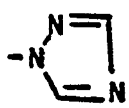 | 152/0,1 |
| 8 | 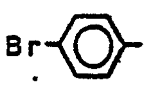 | 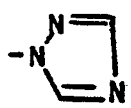 | 180/0,1 |
| 9 | 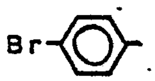 | 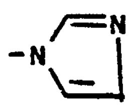 | 155/0,1 |

Anwendungsbeispiele

In dem folgenden Anwendungsbeispiel wird die nachstehend aufgeführte bekannte Verbindung als Vergleichssubstanz verwendet:

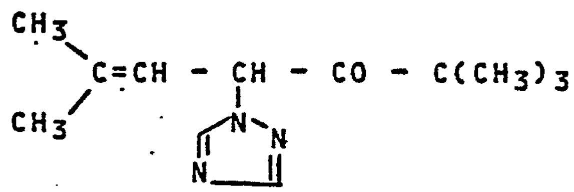

(A)

2,6,6-Trimethyl-4-(1,2,4-triazol-1-yl)-hepten-5-on.

Beispiel A

Pyrenophora teres-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 2, 3 und 4.

Patentansprüche

1. 1-Aryl-3-azolyl-4,4-dimethyl-1-pentene der Formel (I),

$$\underset{\displaystyle Az}{Ar-CH=CH-CH}-C(CH_3)_3 \qquad (I)$$

in welcher

Ar für gegebenenfalls substituiertes Aryl steht und

Az für 1,2,4-Triazol-1-yl oder Imidazol-1-yl steht,

und deren pflanzenverträgliche Säureadditionssalze und Metallsalz-Komplexe.

2. 1-Aryl-3-azolyl-4,4-dimethyl-1-pentene der Formel (I) in Anspruch 1, wobei

Az für 1,2,4-Triazol-1-yl oder Imidazol-1-yl steht und

Ar für gegebenenfalls einfach oder mehrfach, gleich oder verschieden substituiertes Aryl mit 6 bis 10 Kohlenstoffatomen steht, wobei als Substituenten in Frage kommen: Halogen, Cyano, Alkyl, Alkoxy, Alkylthio, Mono- und Dialkylamino mit

jeweils 1 bis 4 Kohlenstoffatomen in den jeweiligen Alkylteilen, Dioxyalkylen mit 1 bis 2 Kohlenstoffatomen, sowie gegebenenfalls durch Alkyl mit 1 bis 4 Kohlenstoffatomen substituiertes 5- oder 6-gliedriges Heteroaryl mit 1 bis 3 Heteroatomen.

3. 1-Aryl-3-azolyl-4,4-dimethyl-1-pentene der Formel (I) in Anspruch 1, wobei

Az für 1,2,4-Triazol-1-yl oder Imidazol-1-yl steht und

Ar für gegebenenfalls einfach oder mehrfach, gleich oder verschieden substituiertes Aryl mit 6 bis 10 Kohlenstoffatomen steht, wobei als Substituenten in Frage kommen: Halogen, Cyano, Alkyl, Alkoxy, Alkylthio, Mono- und Dialkylamino mit jeweils 1 bis 4 Kohlenstoffatomen in den jeweiligen Alkylteilen, Dioxyalkylen mit 1 bis 2 Kohlenstoffatomen, sowie gegebenenfalls durch Alkyl mit 1 bis 4 Kohlenstoffatomen substituiertes 5- oder 6-gliedriges Heteroaryl mit 1 bis 3 Stickstoff-, Sauerstoff- oder Schwefelatomen.

4. 1-Aryl-3-azolyl-4,4-dimethyl-1-pentene der Formel (I) in Anspruch 1, wobei

Az für 1,2,4-Triazol-1-yl oder Imidazol-1-yl steht und

Ar für gegebenenfalls ein- bis dreifach, gleich oder verschieden substituiertes Phenyl oder Naphthyl steht, wobei als Substituenten in Frage kommen: Fluor, Chlor, Brom, Methyl, Ethyl, Methoxy, Methylthio, Methylamino und Dimethylamino, Dioxymethylen oder durch Methyl und/oder Ethyl substituiertes 1,2,3-Triazol-2-yl.

5. Verfahren zur Herstellung von 1-Aryl-3-azolyl-4,4-dimethyl-1-pentenen der Formel (I)

$$Ar-CH=CH-\underset{Az}{CH}-C(CH_3)_3 \qquad (I)$$

in welcher

Ar für gegebenenfalls substituiertes Aryl steht und

Az für 1,2,4-Triazol-1-yl oder Imidazol-1-yl steht,

dadurch gekennzeichnet, daß man 1-Aryl-3-halogen-4,4-dimethyl-1-pentene der allgemeinen Formel (II)

$$Ar-CH=CH-\underset{Hal}{CH}-C(CH_3)_3 \qquad (II)$$

in welcher

Ar die oben angegebene Bedeutung hat und
Hal für Halogen steht,

mit 1,2,4-Triazol oder Imidazol gegebenenfalls in Gegenwart eines Verdünnungsmittels und gegebenenfalls in Gegenwart eines Säurebindemittels umsetzt und gegebenenfalls an die so erhaltenen Verbindungen der allgemeinen Formel (I) eine Säure oder ein Metallsalz addiert.

6. Pflanzenschutzmittel, gekennzeichnet durch einen Gehalt an mindestens einem 1-Aryl-3-azolyl-4,4-dimethyl-1-penten der Formel (I) in Ansprüchen 1 und 5.

7. Verfahren zur Bekämpfung von Pilzen, dadurch gekennzeichnet, daß man 1-Aryl-3-azolyl-4,4-dimethyl-1-pentene der Formel (I) in Ansprüchen 1 und 5 auf Pilze oder ihren Lebensraum einwirken läßt.

8. Verwendung von 1-Aryl-3-azolyl-4,4-dimethyl-1-pentenen der Formel (I) in Ansprüchen 1 und 5 als Pflanzenschutzmittel.

9. Verwendung von 1-Aryl-3-azolyl-4,4-dimethyl-1-pentenen der Formel (I) in Ansprüchen 1 und 5 zur Bekämpfung von Pilzen.

10. Verfahren zur Herstellung von Pflanzenschutzmitteln, dadurch gekennzeichnet, daß man 1-Aryl-3-azolyl-4,4-dimethyl-1-pentene der Formel (I) in Ansprüchen 1 und 5 mit Streckmitteln und/oder oberflächenaktiven Mitteln vermischt.